# EUROPEAN PATENT SPECIFICATION

(11) **EP 2 839 810 B1**
(45) Date of publication and mention of the grant of the patent: **06.04.2016**
(21) Application number: 14158181.9
(22) Date of filing: 06.07.2012
(51) Int. Cl.: A61F 2/30, A61F 2/38

(54) **Apparatus for augmenting the posterior aspect of a femoral knee joint prosthesis component**
Vorrichtung zur Vergrösserung der Hinteransicht einer Oberschenkel-Kniegelenkprothesenkomponente
Appareil pour augmenter l'aspect postérieur d'un composant de prothèse d'articulation fémorale du genou

(30) Priority: 07.07.2011 US 201161505310 P
(43) Date of publication of application: 25.02.2015
(62) Divisional of application: 12743576.6
(73) Proprietor: Zimmer, Inc., Warsaw IN 46580 (US)
(72) Inventor: Metzger, Dianne S., Huntington, IN 46750 (US); Vaughan, Ian R., Fort Wayne, IN 46814 (US); Todd, Dwight T., Columbia City, IN 46725 (US)
(74) Representative: Mays, Julie

(56) References cited:
- EP-A1- 0 378 928
- EP-A2- 0 745 361
- US-A- 5 370 693
- US-A1- 2005 149 052

## Description

### BACKGROUND

### 1. Field of the Disclosure.

The present disclosure relates generally to a femoral prosthesis component. More particularly, the present disclosure relates to an apparatus for augmenting the posterior aspect of a femoral knee joint prosthesis component and a method of manufacturing the same.

### 2. Description of the Related Art.

Knee replacement surgery methods and knee joint prostheses are known in the art. A typical total knee joint prosthesis includes a tibial component that is attached to a proximal portion of a tibia, and a femoral component that is attached to a distal portion of a femur. The femoral component rides on the exposed surface of the tibia component, replicating knee movement and providing articulation similar to the natural, anatomical articulation of the knee joint. When knee replacement surgery is performed, an incision is made to expose the knee joint in order to enable removal of both the proximal portion of the tibia and the distal portion of the femur, which creates surfaces upon which the tibial component and the femoral component of the knee prosthesis can be attached.

In certain situations, the surgeon must remove more of the distal femur than desired, because of disease or trauma. Consequently, there are times when a femoral component selected by a surgeon to provide good joint kinematics once knee arthroplasty is completed has a distance spanning the anterior bone contacting surface to the posterior bone contacting surface that is greater than a distance spanning an anterior prepared distal femur surface to a posterior prepared distal femur surface. In these situations, femoral prosthesis augments can be utilized to provide structural support and to prevent gaps between the distal femur and the femoral component of the knee prosthesis installed on the distal femur.

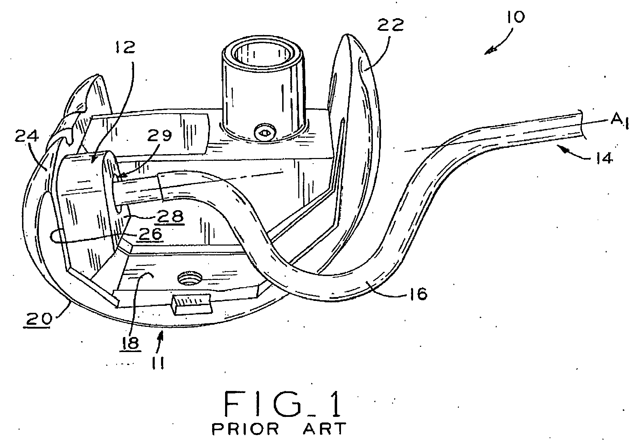

An existing femoral knee prosthesis system including a femoral augment, and method of use are shown in the "Zimmer® NexGen® Trabecular Metal Augments, Abbreviated Surgical Technique" brochure, copyright 2004, 2006, published by Zimmer, Inc. Fig. 1 is representative of a femoral knee prosthesis system utilized with the above surgical technique. Referring to Fig. 1, femoral knee prosthesis system 10 includes femoral component 11, femoral posterior augment 12, and offset drive shaft 16. Such a system is disclosed in US 2005/0149052A1.

Femoral component 11 generally includes bone contacting surface 18, opposing articular surface 20, anterior flange 22, and posterior side 24 opposite anterior flange 22. Further, femoral component 11 has a bore (not shown) located in posterior side 24. The bore (not shown) located in posterior side 24 has a longitudinal axis A₁ which intersects anterior flange 22.

Femoral posterior augment 12 generally includes femoral component contacting surface 26, opposing outer surface 28, and augment cavity 29 spanning femoral component contacting surface 26 and outer surface 28. With augment 12 positioned adjacent posterior side 24 of femoral component 11 and augment cavity 29 aligned with the bore (not shown) located in posterior side 24 of femoral component 11, augment cavity 29 has a longitudinal axis that is collinear with longitudinal axis A₁ of the bore (not shown) located in posterior side 24 and which also intersects anterior flange 22.

Referring to Fig. 1, proximal end 14 of offset drive shaft 16 is operably connected to a torque wrench (not shown) and used to tighten a fastener (not shown) into the bore (not shown) located in posterior side 24 of femoral component 11 to secure femoral posterior augment 12 to posterior side 24 of femoral component 11. Femoral knee prosthesis system 10 requires offset drive shaft 16 to avoid anterior flange 22 of femoral component 11 when securing augment 12 to femoral component 11. However, offset drive shaft 16 of femoral knee prosthesis system 10 cannot be rotated through 360 degrees because anterior flange 22 provides a physical barrier preventing drive shaft 16 from continuous rotation. In femoral knee prosthesis system 10, a straight drive shaft cannot be utilized to tighten a fastener to secure posterior augment 12 to posterior side 24 of femoral component 11 because anterior flange 22 provides a physical barrier which blocks a straight drive shaft from being used.

### SUMMARY

The present invention is defined in claims 1 and 11 and provides a femoral component having an anterior flange and a condyle opposite the anterior flange. The condyle can be augmented by a femoral posterior augment. The femoral posterior augment is securable to the condyle by a fastener which is actuated to a securement position by a rotatable driver which does not contact the anterior flange through rotation of the driver sufficient to actuate the fastener to secure the femoral augment to the condyle.

In one embodiment, the present invention provides a femoral component having a condyle with a bore formed therein, the bore having a longitudinal axis which does not intersect the anterior flange of the femoral component, and a femoral posterior augment having a cavity complementary to the bore of the femoral component. The femoral component and the femoral posterior augment in accordance with embodiments of the present invention allows a fastener to be placed in both the cavity of the femoral posterior augment and the bore of the femoral component and allows a tool, such as a torque wrench, having a straight driver to be used to secure the femoral posterior augment to a posterior facet of the femoral component. Because the longitudinal axis of the bore does not intersect the anterior flange, an offset driver is not needed to secure the posterior augment to the posterior facet of the femoral component because the anterior flange does not have to be avoided when securing the posterior augment to the posterior facet of the femoral component. The straight driver that may be used in accordance with one form of the present invention has a linear longitudinal axis and is capable of transferring an impaction force from one end to another end of the driver along its longitudinal axis and is further capable of transferring torque from the one end to the other end about its longitudinal axis.

In one embodiment, the fastener of the present invention may include a set screw which traverses a cavity of the posterior augment and the bore of the femoral component to secure the posterior augment to the condyle of the femoral component. In another embodiment, the fastener of the present invention may include a set screw and an expansion collet. In one embodiment, the expansion collet may extend from the augment and with the expansion collet of the augment aligned in the bore of the femoral component, the set screw may traverse the cavity of the posterior augment and the bore of the femoral component to engage the expansion collet with a securement feature of the condyle to secure the posterior augment to the condyle. In an alternative embodiment, the expansion collet may extend from the condyle of the femoral component and with the expansion collet of the condyle aligned in the cavity of the posterior augment, the set screw may traverse the cavity of the posterior augment and the bore of the femoral component to engage the expansion collet with a securement feature of the posterior augment to secure the posterior augment to the condyle.

In one embodiment, the present invention provides a femoral knee prosthesis component including an anterior flange, the anterior flange having an anterior flange bone contacting surface comprising an anterior facet, a condyle extending from the anterior flange, the condyle having a condyle bone contacting surface comprising a facet opposing the anterior facet, the condyle having a condyle bore formed therein, the condyle bore defining a condyle bore longitudinal axis that intersects the facet and does not intersect the anterior flange. In an embodiment of the present invention, the condyle bore longitudinal axis intersects the facet at an intersection point, the intersection point positioned such that an axis perpendicular to the facet and intersecting the intersection point may have a minimum spacing from the anterior flange of no more than 8 mm. In further embodiments the minimum space may be no more than 5 mm. In one embodiment, the condyle bore longitudinal axis may not be perpendicular to the condyle bone contacting surface. In one form of the present invention, the femoral knee prosthesis component may be provided in combination with a posterior augment for securement to the condyle and a fastener operable to secure the augment to the condyle. In further embodiments, the combination may include a driver, the driver comprising a handle and a drive shaft extending from the handle and terminating in a drive end, the drive shaft defining a longitudinal axis, the drive end mateable with the fastener, the drive shaft defining a drive shaft radius from the longitudinal axis of the drive shaft to a perimeter extent of the drive shaft, with the drive shaft positioned to drive the fastener into an engaged position to secure the augment to the condyle, the driver is rotatable through a rotation about the longitudinal axis of the drive shaft sufficient to actuate the fastener to secure the augment to the condyle and the longitudinal axis of the drive shaft is positioned a minimum distance from the anterior flange throughout the rotation about the longitudinal axis of the drive shaft sufficient to actuate the driver to secure the augment to the condyle, the minimum distance at least equal to the drive shaft radius, whereby the drive shaft does not contact the anterior flange through the rotation about the longitudinal axis of the drive shaft sufficient to actuate the fastener to secure the augment to the condyle.

The present invention, in a further form thereof, comprises a femoral knee prosthesis component including an anterior flange, the anterior flange having an anterior flange bone contacting surface comprising an anterior facet, a condyle extending from the anterior flange, the condyle having a condyle bone contacting surface comprising a facet opposing the anterior facet, the condyle having a condyle bore formed therein, the condyle bore defining a condyle bore longitudinal axis that intersects the facet and does not intersect the anterior flange, an augment for securement to the condyle, the augment including an augment bore formed therethrough, and a fastener sized and shaped to traverse the augment bore and secure the augment to the condyle.

The present invention, in another form thereof, comprises, in combination, a femoral knee prosthesis component including an anterior flange, the anterior flange having an anterior flange bone contacting surface. The femoral knee prosthesis component of this form of the present invention further includes a condyle extending from the anterior flange, the condyle having a condyle bone contacting surface, the condyle bone contacting surface opposing the anterior flange bone contacting surface, the condyle having a condyle bore formed therein, the condyle bore defining a condyle bore longitudinal axis which does not intersect the anterior flange. The combination of this form of the present invention may further include a posterior augment for securement to the condyle, the posterior augment having an augment bore formed therethrough, the augment bore defining an augment bore longitudinal axis, the condyle including a securement feature extending into the condyle bore. The combination of this form of the present invention may additionally include a fastener, the fastener traversing the augment bore and the condyle bore to engage the securement feature and secure the augment to the condyle, with the condyle bore longitudinal axis aligned with the augment bore longitudinal axis. The combination of this form of the present invention may further include a driver including a handle and a drive shaft extending from the handle and terminating in a drive end, the drive end matable with the fastener, the drive shaft defining a drive shaft radius to a perimeter extent of the drive shaft, the condyle bore longitudinal axis spaced a minimum distance from the anterior flange bone contacting surface, the minimum distance at least equal to the drive shaft radius. In one exemplary embodiment, the minimum distance may be 4 mm. In one embodiment, the anterior flange bone contacting surface comprises an anterior facet and the minimum distance is measured in a plane including the anterior facet.

The present invention, in a further form thereof, comprises a femoral prosthesis system including a femoral knee prosthesis component having an anterior flange, the anterior flange having an anterior flange bone contacting surface, and a condyle extending from the anterior flange, the condyle having a condyle bone contacting surface opposing the anterior flange bone contacting surface. The femoral prosthesis system of this form of the present invention may further include an augment for securement to the condyle. The femoral prosthesis system of this form of the present invention may further include a fastener operable to secure the augment to the condyle, the fastener defining a fastener longitudinal axis, with the fastener positioned to secure the augment to the condyle, the fastener longitudinal axis does not intersect the anterior flange.

The present invention, in another form thereof, comprises, in combination, a femoral prosthesis system including a femoral knee prosthesis component including an anterior flange, the anterior flange having an anterior flange bone contacting surface, and a condyle extending from the anterior flange, the condyle having a condyle bone contacting surface, the condyle bone contacting surface opposing the anterior flange bone contacting surface. The combination of this form of the present invention may further include an augment for securement to the condyle. The combination of this form of the present invention may additionally include a fastener operable to secure the augment to the condyle. The combination of this form of the present invention may further include a driver including a handle and a drive shaft extending from the handle and terminating in a drive end, the drive shaft defining a longitudinal axis, the drive end matable with the fastener, the drive shaft defining a drive shaft radius from the longitudinal axis of the drive shaft to a perimeter extent of the drive shaft, with the drive shaft positioned to drive the fastener into an engaged position to secure the augment to the condyle, the driver is rotatable through a rotation about the longitudinal axis of the drive shaft sufficient to actuate the fastener to secure the posterior augment to the condyle and the longitudinal axis of the drive shaft is positioned a minimum distance from the anterior flange throughout the rotation about the longitudinal axis of the drive shaft sufficient to actuate the fastener to secure the posterior augment to the condyle, the minimum distance at least equal to the drive shaft radius, whereby the drive shaft does not contact the anterior flange through the rotation about the longitudinal axis of the drive shaft sufficient to actuate the fastener to secure the posterior augment to the condyle. In one exemplary embodiment, the minimum distance may be 4 mm.

The present invention, in yet another form thereof, comprises a method of manufacturing a femoral prosthesis component useable with a posterior augment, the method including: forming a femoral prosthesis component of a biologically compatible material, the femoral prosthesis component including an anterior flange having an anterior flange bone contacting surface; and a condyle extending from the anterior flange, the condyle having a condyle bone contacting surface, the condyle bone contacting surface opposing the anterior flange bone contacting surface; selecting a rotary forming tool, the rotary forming tool having a drive shaft with a drive shaft periphery and a longitudinal axis, the drive shaft defining a drive shaft radius to the drive shaft periphery; positioning the rotary forming tool such that the longitudinal axis of the drive shaft of the rotary forming tool intersects the condyle bone contacting surface of the condyle at an intersection point and is oblique to the condyle bone contacting surface and the longitudinal axis of the drive shaft of the rotary forming tool is spaced a minimum distance from the anterior flange bone contacting surface, the minimum distance at least equal to the drive shaft radius, wherein, with the rotary forming tool longitudinal axis intersecting the intersection point, the anterior flange presents a physical barrier preventing rotary forming tool from being positioned perpendicular to the condyle bone contacting surface; and, with the rotary forming tool positioned as defined in the positioning step, actuating the rotary forming tool to form a bore through the condyle bone contacting surface and into the condyle.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
Fig. 1 is a perspective view of a prior art femoral knee prosthesis system including a femoral component, a femoral posterior augment, and an offset driver;
Fig. 2 is a perspective view of a femoral component in accordance with the present disclosure;
Fig. 3 is a perspective view of a femoral posterior augment in accordance with the present disclosure;
Fig. 4 is an exploded perspective view of a femoral knee prosthesis system in accordance with the present disclosure;
Fig. 5 is a partial sectional, plan view of a femoral knee prosthesis system in accordance with the present disclosure with a posterior side of a femoral component and a femoral posterior augment shown in section;
Fig. 6A is a fragmentary, cross-sectional view of the posterior side of the femoral component of Fig. 5 and the femoral posterior augment of Fig. 5, illustrating collet fingers of the femoral posterior augment in a non-expanded position;
Fig. 6B is a fragmentary, cross-sectional view of the posterior side of the femoral component of Fig. 5 and the femoral posterior augment of Fig. 5, illustrating collet fingers of the femoral posterior augment in an expanded position;
Fig. 7A is a perspective view of a femoral knee prosthesis system including a femoral distal augment and a femoral posterior augment in accordance with the present disclosure;
Fig. 7B is a cross-sectional view taken along line 7B-7B of Fig. 7A;
Fig. 8 is a partial sectional, plan view of a femoral knee prosthesis system in accordance with another exemplary embodiment of the present disclosure with a posterior side of a femoral component and a femoral posterior augment shown in section;
Fig. 9A is a fragmentary, cross-sectional view of a posterior side of a femoral component and a femoral posterior augment of a femoral knee prosthesis system in accordance with another exemplary embodiment of the present disclosure, illustrating collet fingers of the femoral component in a non-expanded position;
Fig. 9B is a fragmentary, cross-sectional view of the posterior side of the femoral component of Fig. 9A and the femoral posterior augment of the Fig. 9A, illustrating collet fingers of the femoral component in an expanded position; and
Fig. 10 is a perspective view illustrating a method of manufacturing a femoral prosthesis component useable with a posterior augment in accordance with the present disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

In the following discussion, "proximal" refers to a direction generally toward the heart of a patient, and "distal" refers to the opposite direction of proximal, i.e., away from the heart of a patient. As used herein, "anterior" refers to a direction generally toward the front of a patient, and "posterior" refers to the opposite direction of anterior, i.e., toward the back of a patient. Further, as used herein, "medial" refers to a direction generally toward the middle of a patient, and "lateral" refers to the opposite direction of medial, i.e., toward the side of a patient. While the exemplary embodiments detailed herein are shown and described with regard to a left knee, it will be appreciated that the present disclosure is equally applicable to a right knee configuration.

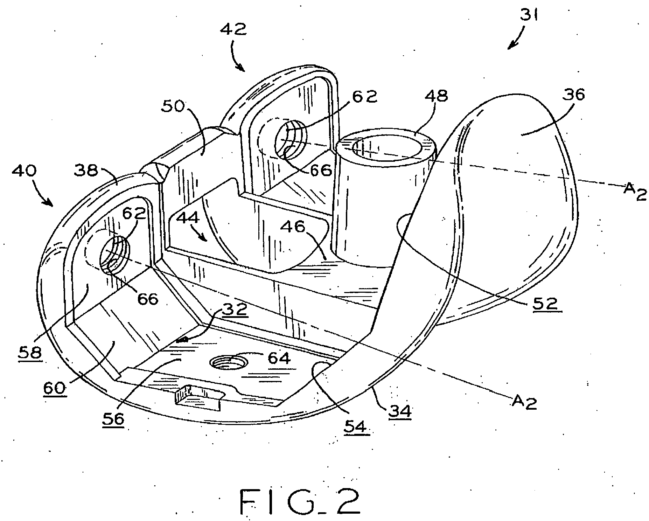

Fig. 2 illustrates a femoral component according to an exemplary embodiment of the present disclosure. Femoral component 31 of the present disclosure is adapted to mount to a distal femur. In the exemplary embodiment of Fig. 2, femoral component 31 is a posterior stabilized femoral component, though it is contemplated that other femoral components may be utilized in accordance with the present disclosure such as femoral components which cooperate to form a cruciate retaining prosthesis or a knee prosthesis having an intermediate level of constraint between a cruciate retaining and posterior stabilized prosthesis. Femoral component 31 may also be made available in a variety of shapes and sizes to accommodate a variety of differing knee physiologies.

Femoral component 31 generally includes bone contacting interior surface 32 and opposing articular exterior surface 34, each extending between anterior flange 36 and posterior side 38. Bone contacting surface 32 is adapted to affix femoral component 31 to a distal portion of a femur, such as with bone cement and/or porous bone-ingrowth material. Femoral component 31 also includes medial condyle 40 and lateral condyle 42, with intercondylar fossa 44 formed between condyles 40, 42. Bone contacting surface 32 of condyles 40, 42 at posterior side 38 opposes anterior flange 36. Condyles 40, 42 are joined by intercondylar box 46. Stem 48 extends proximally from intercondylar box 46. Articular exterior surface 34 is disposed generally opposite bone contacting interior surface 32, and is comprised of the exterior surfaces of medial and lateral condyles 40, 42 as well as the exterior surface of anterior flange 36. Femoral component 31 also includes femoral cam 50 formed at posterior side 38. Femoral cam 50 spans medial and lateral condyles 40, 42.

In a posterior stabilized femoral component, such as femoral component 31, cam 50 cooperates with a spine (not shown) formed in a tibial component (not shown) to guide or constrain motion within certain predefined boundaries. Posterior stabilized prostheses are appropriate where the posterior cruciate ligament (PCL) is torn or otherwise damaged, or where the PCL is resected during surgery.

Bone contacting interior surface 32 of femoral component 31 is adapted to mate with a resected articular surface of a distal femur (not shown) and includes anterior facet 52, anterior chamfer facet 54, distal facet 56, posterior facet 58, and posterior chamfer facet 60. Anterior facet 52, anterior chamfer facet 54, posterior facet 58, and posterior chamfer facet 60 correspond to the cuts made to a distal end of a femur to allow implantation of a femoral prosthesis component, i.e., an anterior cut, an anterior chamfer cut, a posterior cut, and a posterior chamfer cut. Anterior facet 52 and posterior facet 58 diverge from each other in a distal to proximal direction to facilitate a surgical technique in which femoral component 11 can be implanted on a femur in a distal to proximal direction. In one exemplary embodiment, anterior facet 52 diverges from posterior facet 58 such that a plane containing anterior facet 52 forms an angle of 1-5° with the plane containing posterior facet 58. Femoral component 31 may be part of a provisional prosthesis system or a final prosthesis system, i.e., femoral component 31 is the final femoral component implanted to a resected distal femur.

Referring to Fig. 2, femoral component 31 includes bores 62 formed in posterior facet 58 on medial condyle 40 and lateral condyle 42. While described with reference to posterior facet 58, the securement feature of the present disclosure may also be formed in posterior chamfer facet 60. Generally, the present invention is employed with a facet of the femoral component that opposes another feature of the femoral component such that the feature presents a barrier to a tool accessing the securement feature along a trajectory perpendicular to the facet with which the securement feature is associated. A bore wall 66 defines each bore 62 having longitudinal axis A₂. Bores 62 are blind bores, i.e., they do not extend completely from posterior facet 58 through to articular exterior surface 34. Longitudinal axis A₂ of bore 62 does not intersect anterior flange 36. In the embodiment illustrated in Fig. 2, posterior facet 58 and anterior facet 52 are generally parallel and longitudinal axis A₂ is not perpendicular to posterior facet 58. As will be understood by the drawings and the description herein, longitudinal axis A₂ represents an imaginary line extending beyond the limits of bore 62. In this embodiment, longitudinal axis A₂ of bore 62 is oblique to posterior facet 58 and anterior facet 52, i.e., longitudinal axis A₂ of bore 62 is neither parallel nor perpendicular to posterior facet 58 and anterior facet 52. In other embodiments, posterior facet 58 of femoral component 31 may be canted relative to anterior facet 52, i.e., posterior facet 58 may not be parallel to anterior facet 52.

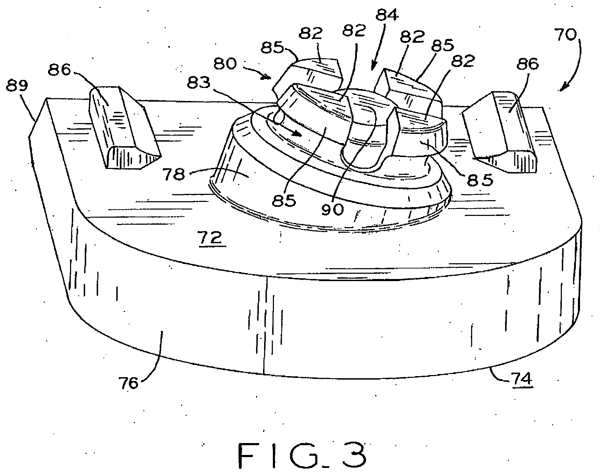

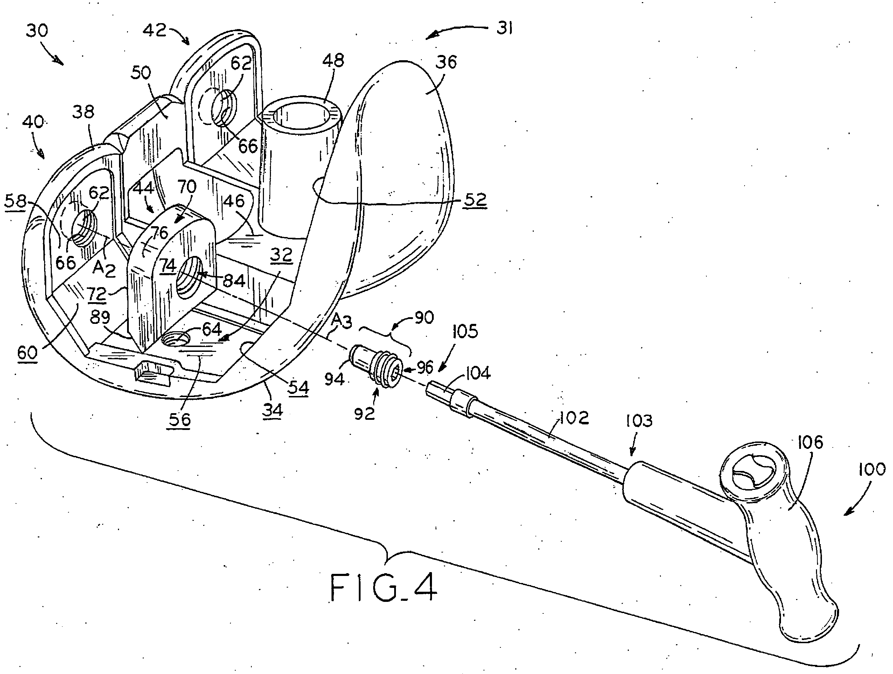

Advantageously, femoral component 31 allows a tool, such as a torque wrench, having a straight driver (such as straight drive shaft 102 shown in Figs. 4, 5, and 8) to be used to secure a femoral posterior augment (such as posterior augment 70 shown in Figs. 3, 4, 5, and 8) to posterior facet 58 of femoral component 31. A straight driver such as straight drive shaft 102 includes a linear longitudinal axis from one end (such as first end 103 shown in Figs. 4, 5, and 8) to another end (such as second end 105 shown in Figs. 4, 5, and 8). Straight drive shaft 102 is capable of transferring an impaction force from first end 103 (shown in Figs. 4, 5, and 8) to second end 105 (shown in Figs. 4, 5, and 8) along longitudinal axis A₂ and is capable of imparting torque from first end 103 to second end 105 about longitudinal axis A₂.

By having bores 62 with longitudinal axes A₂ which do not intersect anterior flange 36, an offset driver (such as offset drive shaft 16 shown in Fig. 1) is not needed to secure posterior augment 70 to posterior facet 58 of femoral component 31 because anterior flange 36 does not have to be avoided when securing posterior augment 70 to posterior facet 58 of femoral component 31.

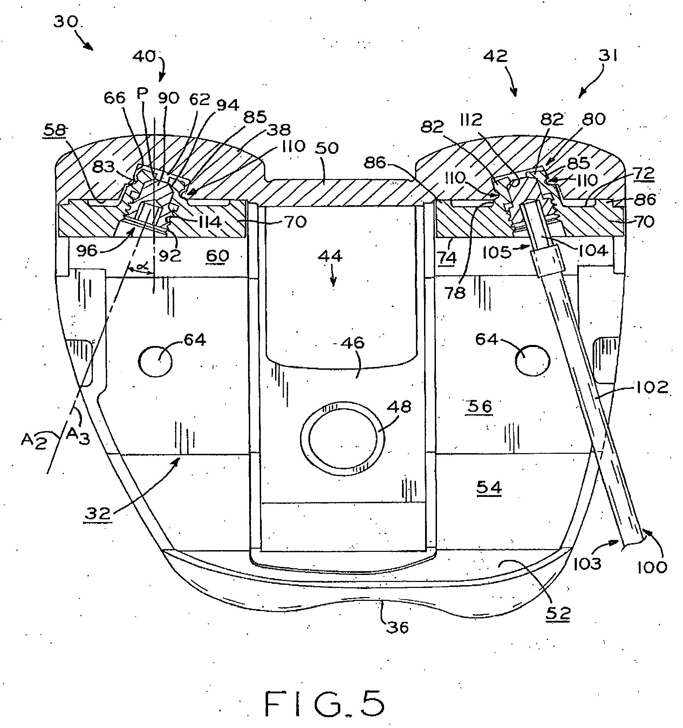

Fig. 5 illustrates perpendicular P which intersects longitudinal axis A₂ of bore 62 at a plane containing posterior facet 58. Perpendicular P is perpendicular to posterior facet 58 and, in certain embodiments, intersects anterior flange 36. In alternative embodiments of the present invention, perpendicular P has a minimum spacing from anterior flange 36 of no more than 8 mm. In further embodiments, perpendicular P has a minimum spacing from anterior flange 36 of no more than 5 mm. Defining perpendicular P as having a minimum spacing from anterior flange 36 of no more than 8 mm or no more than 5 mm is meant to be inclusive of embodiments in which perpendicular P intersects anterior flange 36, i.e., has a minimum spacing of zero. That is, "not more than" is inclusive of any value including to or less than the stated value. In this context "minimum spacing" denotes the shortest distance between perpendicular P and anterior flange 36. Through this document phrases such as "minimum spacing" and "minimum distance" denote the shortest distance between two items, such as a physical structure of the prosthesis and an imaginary line or axis. In one exemplary embodiment, condyle bore longitudinal axis A₂ forms an angle α with perpendicular P in the range of 7-27°. In an exemplary embodiment the angle α is in the range of 12-22°. In a further exemplary embodiment the angle α equals 17°. In the illustrated embodiment, angle α is measured in a transverse anatomic plane. In certain embodiments, angle α will be measured in a plane parallel to distal facet 56.

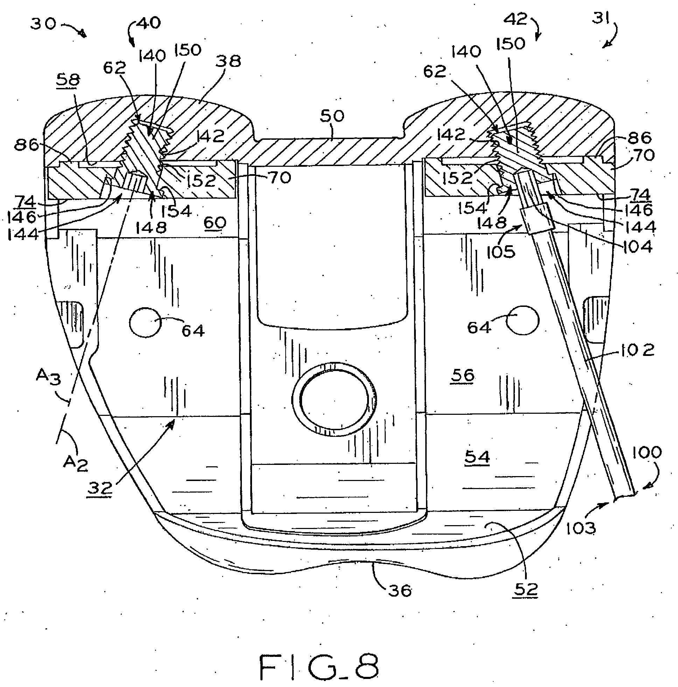

Referring to Figs. 5 and 8, longitudinal axis A₂ of bore 62 does not intersect anterior flange 36 and longitudinal axis A₂ of bore 62 is spaced from anterior flange 36 a minimum distance. The minimum distance is at least as great as the extent of straight drive shaft 102 from its longitudinal axis to allow straight drive shaft 102 to avoid anterior flange 36 when straight drive shaft 102 is used to effect securement of posterior augment 70 to posterior side 38 of femoral component 31. In an exemplary embodiment, the drive shaft is a cylindrical drive shaft of having a radius of about 4 mm. In such an embodiment, the minimum distance between longitudinal axis A₂ of bore 62 and anterior flange 36 is 4 mm. In the embodiment illustrated in Figs. 2, 5 and 8, the minimum distance is measured in a plane including anterior facet 52. With set screw 90 or fastener 140 in position to be secured to attach posterior augment 70 to posterior side 38 of femoral component 31, the portion of drive shaft 102 adjacent anterior flange 36 during the act of securement, i.e., until set screw 90 or fastener 140 is fastened in bore 62, is spaced a distance that is at least as great as the extent of drive shaft 102 from its longitudinal axis. In one embodiment, the entire drive shaft 102 is spaced from anterior flange 36 a distance that is at least as great as the extent of drive shaft 102 from its longitudinal axis.

In the embodiment illustrated in Figs. 5 and 8, straight drive shaft 102 has a cylindrical cross-sectional shape. In such an embodiment, longitudinal axis A₂ of bore 62 is spaced from anterior flange 36 by at least the radius of straight drive shaft 102. In other embodiments, drive shaft 102 can have multi-sided polygon cross-sectional shapes, such as square or rectangular cross-sectional shapes. In such embodiments, a perimeter extent of drive shaft 102 is the radially outward portion farthest from the longitudinal axis of drive shaft 102, and the cross-section of drive shaft 102 defines a drive shaft radius to the perimeter extent of drive shaft 102. In these embodiments, longitudinal axis A₂ of bore 62 is spaced from anterior flange 36 by at least the radius of drive shaft 102. In alternative embodiments, drive shaft 102 can have an irregular shape, e.g., drive shaft 102 could have an inconsistent cross-section along its length and/or have a shaft center through certain cross-sections that is not always coincident with the longitudinal axis of the drive shaft. In embodiments including drive shafts having irregular shapes, the drive shaft could have perimeter extents a varying distance from the longitudinal axis of the drive shaft. In such embodiments, longitudinal axis A₂ of bore 62 is spaced from anterior flange 36 by at least the distance between the longitudinal axis of the drive shaft and the perimeter extent of the drive shaft which will be adjacent to anterior flange 36 in use to secure posterior augment 70 to posterior side 38 of femoral component 31. In this way, drive shaft 102 will, in use, be rotatable about its longitudinal axis through 360 degrees of rotation without engaging anterior flange 36. In certain embodiments, rotation of drive shaft through 360° of rotation will not be required to actuate a fastener to secure a posterior augment to the condyle. In any event, the embodiments of the present disclosure will allow sufficient rotation of a driver to actuate a fastener to secure a posterior augment to the posterior condyle of a femoral prosthesis.

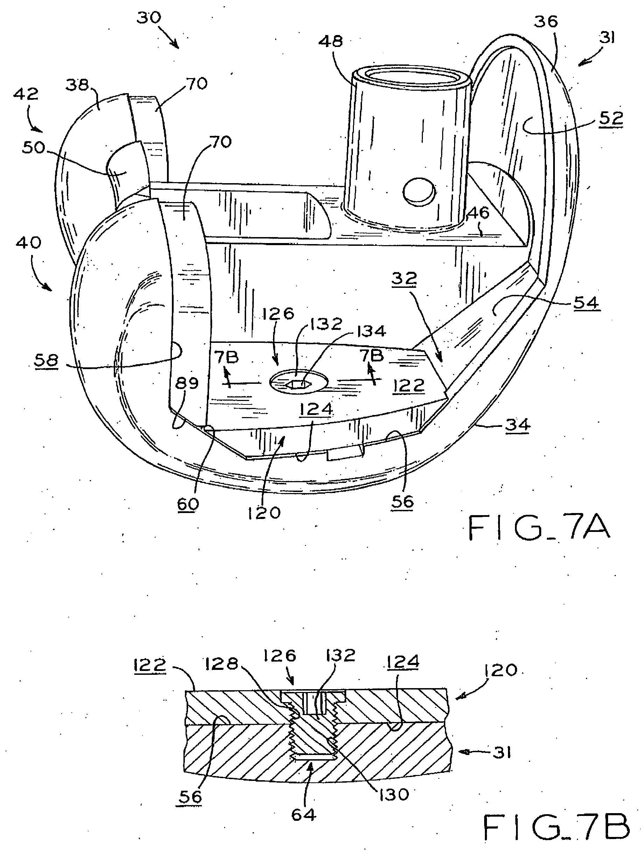

Referring to Figs. 2 and 7A, femoral component 31 also includes distal bore 64 located in distal facet 56 for receiving a fastener to secure a femoral distal augment (such as distal augment 120 shown in Fig. 7A) to distal facet 56 as will be discussed in more detail later.

Femoral component 31 may be constructed of any biocompatible ceramic or metal, including, but not limited to, titanium, a titanium alloy, cobalt chromium, cobalt chromium molybdenum, porous tantalum, or a combination of these materials, for example. Femoral component 31 may include a highly porous biomaterial useful as a bone substitute and as cell and tissue receptive material. A highly porous biomaterial may have a porosity as low as 55%, 65%, or 75% or as high as 80%, 85%, or 90%. An example of such a material is produced using Trabecular Metal™ technology generally available from Zimmer, Inc., of Warsaw, Indiana. Trabecular Metal™ is a registered trademark of Zimmer, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 to Kaplan, the entire disclosure of which is expressly incorporated herein by reference. In addition to tantalum, other metals such as niobium, or alloys of tantalum and niobium with one another or with other metals may also be used.

Generally, the porous tantalum structure includes a large plurality of ligaments defining open spaces therebetween, with each ligament generally including a carbon core covered by a thin film of metal such as tantalum, for example. The open spaces between the ligaments form a matrix of continuous channels having no dead ends, such that growth of cancellous bone through the porous tantalum structure is uninhibited. The porous tantalum may include up to 75%, 85%, or more void space therein. Thus, porous tantalum is a lightweight, strong porous structure which is substantially uniform and consistent in composition, and closely resembles the structure of natural cancellous bone, thereby providing a matrix into which cancellous bone may grow to provide fixation of femoral component 31 to the patient's bone.

The porous tantalum structure may be made in a variety of densities in order to selectively tailor the structure for particular applications. In particular, as discussed in the above-incorporated U.S. Patent No. 5,282,861, the porous tantalum may be fabricated to virtually any desired porosity and pore size, and can thus be matched with the surrounding natural bone in order to provide an improved matrix for bone ingrowth and mineralization.

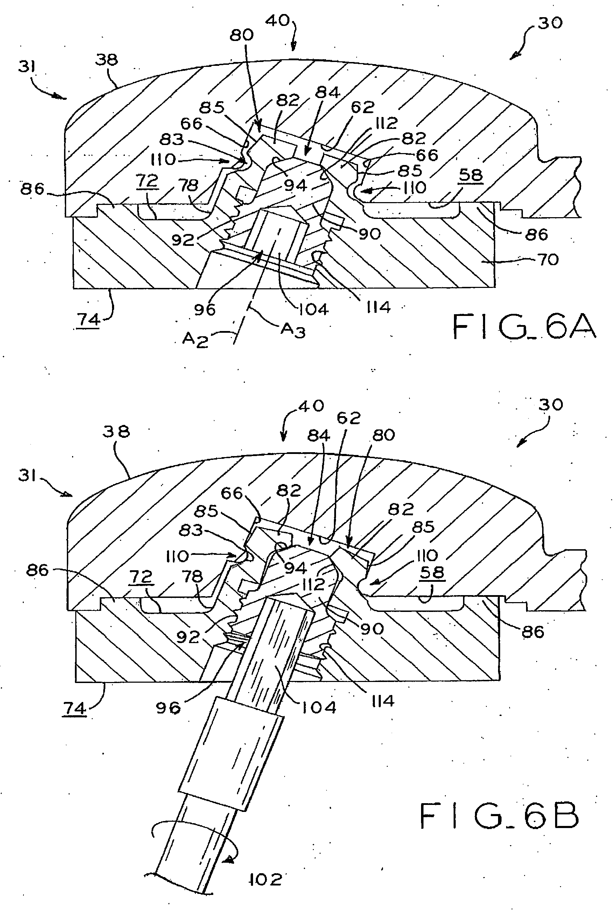

Fig. 3 illustrates features of femoral posterior augment 70 according to an exemplary embodiment of the present disclosure. Posterior augment 70 provides structural support in areas of bone loss of a distal femur and prevents gaps between the distal femur and posterior facet 58 of femoral component 31 installed on the distal femur. Posterior augment 70 generally includes femoral component contacting surface 72, opposing bone contacting surface 74, peripheral wall 76 spanning femoral component contacting surface 72 and bone contacting surface 74, boss 78, and expansion collet 80. Boss 78 extends at an angle from femoral component contacting surface 72 and includes expansion collet 80 at a terminal end thereof. The attachment of posterior augment 70 to femoral component 31 using expansion collet 80 will be discussed in more detail later. Expansion collet 80 generally includes four collet fingers 82 located around a periphery of expansion collet 80. Annular groove 83 is located between collet fingers 82 and boss 78. Collet fingers 82 include tapered exterior portion 85 and tapered interior portion 112 (shown in Figs. 6A and 6B).

Posterior augment 70 also includes augment cavity 84 spanning augment bone contacting surface 74 and the extent of collet fingers 82. Augment cavity 84 includes augment threaded interior portion 114 (shown in Figs. 6A and 6B), which, in use, receives a threaded fastener such as set screw 90. Set screw 90 will be discussed in more detail later. With augment 70 positioned adjacent bore 62 of posterior facet 58 of femoral component 31 such that augment cavity 84 is aligned with bore 62, longitudinal axis A₃ (shown in Figs. 4 and 5) of augment cavity 84 is collinear with longitudinal axis A₂ of bore 62 and does not intersect anterior flange 36.

Referring to Fig. 3, posterior augment 70 further includes two augment protuberances 86 extending from femoral component contacting surface 72. One augment protuberance 86 is located on each side of boss 78 and protuberances 86 can be used to properly index posterior augment 70 relative to posterior facet 58 of femoral component 31. In alternative embodiments, protuberances 86 can have a continuous surface which can fit with an undercut surface of posterior facet 58 of femoral component 31. Referring to Figs. 3 and 4, posterior augment 70 includes beveled edge 89. Beveled edge 89 and femoral component contacting surface 72 mimic the contour of posterior facet 58 and posterior chamfer facet 60 of femoral component 31 to allow for a close fit of femoral component contacting surface 72 of augment 70 to posterior facet 58 of femoral component 31 and beveled edge 89 of augment 70 to posterior chamfer facet 60 of femoral component 31.

Posterior augment 70 may be constructed of any bio-compatible ceramic or metal, including, but not limited to, titanium, a titanium alloy, cobalt chromium, cobalt chromium molybdenum, or porous tantalum, or a combination of the materials, for example. In certain embodiments, the augments of the present disclosure can be made of a Tivanium™ alloy (Ti-6A1-4V) generally available from Zimmer, Inc., of Warsaw, Indiana. Tivanium™ is a registered trademark of Zimmer, Inc. Posterior augment 70 may also be constructed in whole or in part of a highly porous biomaterial such as a material produced using Trabecular Metal™ technology as described above.

Similar to femoral component 31, posterior augment 70 may be part of a provisional prosthesis system or a final prosthesis system. In certain embodiments, posterior augment 70 will be disposable. For example, in a provisional prosthesis system, if posterior augment 70 is deformed in use, posterior augment 70 could be disposed of after its use as a trial.

Figs. 4-6B illustrate attachment of posterior augment 70 to posterior side 38 of femoral component 31. As shown in Fig. 4, a surgical kit in accordance with the present disclosure includes femoral component 31, posterior augment 70, set screw 90, and torque wrench 100 having straight drive shaft 102. Referring to Fig. 4, set screw 90 generally includes threaded portion 92, tapered portion 94, and internal female socket 96. Set screw 90 may be constructed of any biocompatible ceramic or metal, including, but not limited to, titanium or a titanium alloy, such as Tivanium™ available from Zimmer, Inc., for example. Torque wrench 100 generally includes straight drive shaft 102, socket head 104, and handle 106 (Fig. 4). Referring to Fig. 5-6B, protrusion 110 extends from bore wall 66 into bore 62, collet fingers 82 include tapered interior portion 112, and posterior augment 70 includes threaded interior portion 114.

Straight drive shaft 102 is operably connected to torque wrench 100 and is used to secure posterior augment 70 onto femoral component 31. Advantageously, because longitudinal axis A₂ of bore 62 of femoral component 31 does not intersect anterior flange 36, straight drive shaft 102 can be used to attach posterior augment 70 onto posterior side 38 of femoral component 31.

Posterior augment 70 is mounted on posterior side 38 of femoral component 31 such that femoral component contacting surface 72 is positioned adjacent posterior facet 58 of femoral component 31 and augment protuberances 86 contact posterior facet 58 (shown in Figs. 6A and 6B). Once posterior augment 70 is properly aligned with posterior facet 58 such that augment cavity 84 is aligned with bore 62, longitudinal axis A₃ (shown in Figs. 4 and 5) of augment cavity 84 is collinear with longitudinal axis A₂ of bore 62 and does not intersect anterior flange 36.

With cavity 84 of posterior augment 70 positioned adjacent bore 62, expansion collet 80 of posterior augment 70 is inserted into bore 62 such that fingers 82 of expansion collet 80 extend beyond posterior facet 58 of femoral component 31 and into bore 62. Referring to Figs. 5-6B, when collet fingers 82 are inserted into bore 62 of femoral component 31, tapered exterior portion 85 of collet fingers 82 slide over and past protrusion 110 in bore 62 such that collet fingers 82 are located past protrusion 110 and protrusion 110 occupies annular groove 83. Fig. 6A illustrates collet fingers 82 in a non-expanded position, with collet fingers 82 slid past protrusion 110 and annular groove 83 positioned adjacent protrusion 110. Next, set screw 90 can be positioned in augment cavity 84.

Once collet fingers 82 are in the position shown in Fig. 6A and set screw 90 is positioned in augment cavity 84, socket head 104 of straight drive shaft 102 can be inserted into internal female socket 96 of set screw 90, and set screw 90 can be tightened by rotating straight drive shaft 102 and torque wrench 100 using handle 106 (Fig. 4). As set screw 90 is threaded into augment cavity 84, tapered portion 94 of set screw 90 cooperates with tapered interior portion 112 of collet fingers 82 and pushes collet fingers 82 outward until exterior portion 85 of collet fingers 82 contacts bore wall 66 of bore 62 and locks collet fingers 82 over protrusion 110 as shown in Fig. 6B. This configuration ensures that with collet fingers 82 mechanically locked over protrusion 110, posterior augment 70 is secured to posterior facet 58 of femoral component 31, such that, significant relative movement between augment 70 and femoral component 31 is prevented.

In an alternate embodiment, referring to Fig. 8, fastener 140 is utilized to attach posterior augment 70 to posterior side 38 of femoral component 31. As shown in Fig. 8, in such an embodiment, bore 62 includes threaded bore portion 142 and posterior augment 70 includes counterbore 144 forming augment shoulder 146. Fastener 140 generally includes head 148 and shank 150 having threaded portion 152. The transition between head 148 and shank 150 define fastener shoulder 154. Fastener shoulder 154 engages augment shoulder 146 in counterbore 144 of posterior augment 70 to draw posterior augment 70 into tight engagement with femoral component 31 as fastener 140 is threaded into threaded bore portion 142. Augment shoulder 146 of posterior augment 70 is recessed sufficiently below augment bone contacting surface 74 of posterior augment 70 such that head 148 of fastener 140 does not protrude above the plane of augment bone contacting surface 74 when fastener 140 is fully tightened. In the alternate embodiment illustrated in Fig. 8, straight drive shaft 102 can be used to tighten fastener 140 to secure posterior augment 70 onto posterior side 38 of femoral component 31 because longitudinal axis A₂ of bore 62 of femoral component 31 does not intersect anterior flange 36.

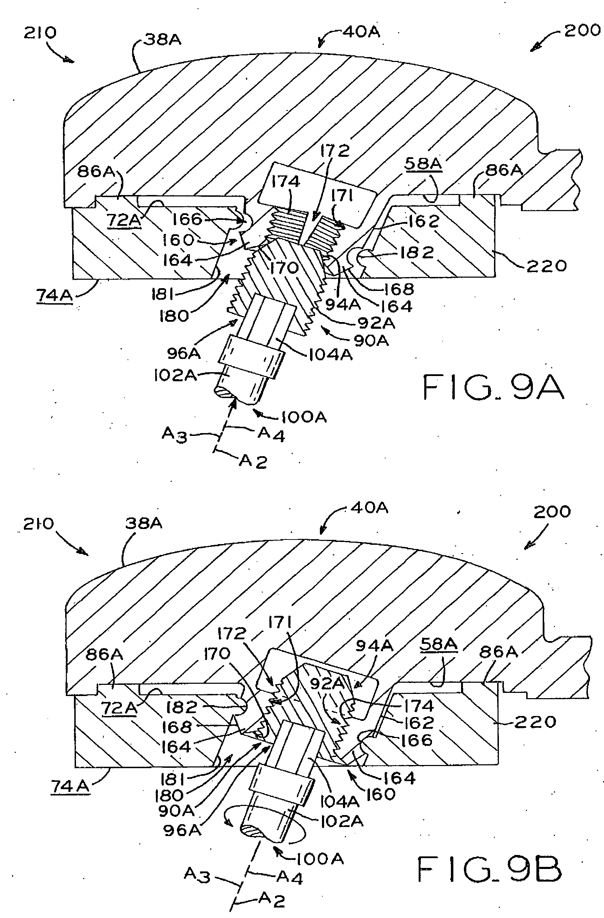

Figs. 9A and 9B illustrate another exemplary embodiment. This embodiment of the present disclosure includes similar features to the embodiment illustrated in Figs. 2-6B. These features are denoted with the same reference number used to identify the corresponding feature in the previous embodiment followed by the letter A. For the sake of brevity, these similar components will not all be discussed in conjunction with the embodiment illustrated in Figs. 9A and 9B.

Referring to Figs. 9A and 9B, femoral knee prosthesis system 200 includes femoral component 210, femoral posterior augment 220, set screw 90A, and torque wrench 100A. In this embodiment, expansion collet 160 extends from condyle 40A of femoral component 210 and condyle boss 162 extends at an angle from posterior facet 58A of femoral component 210 and includes expansion collet 160 at a terminal end thereof. In one embodiment, expansion collet 160 is formed integral to posterior facet 58A of condyle 40A. Expansion collet 160 generally includes collet fingers 164 located around a periphery of expansion collet 160. Annular groove 166 is located between collet fingers 164 and condyle boss 162. Collet fingers 164 include tapered exterior portion 168 and tapered interior portion 170. Expansion collet 160 and condyle boss 162 also include condyle bore wall 171 defining condyle bore 172 having longitudinal axis A₂. Condyle bore 172 spans the extent of collet fingers 164 and condyle boss 162. Longitudinal axis A₂ of condyle bore 172 does not intersect anterior flange 36 (Figs. 2 and 5). Referring to Figs. 9A and 9B, condyle bore 172 penetrates posterior facet 58A of femoral component 210. In other embodiments, no portion of condyle bore 172 penetrates posterior facet 58 of femoral component 210. Condyle bore 172 includes threaded interior portion 174, which, in use, receives a threaded fastener such as set screw 90A.

Referring to Figs. 9A and 9B, posterior augment 220 includes interior bore wall 181 defining augment bore 180 having longitudinal axis A₃. Augment bore 180 spans femoral component contacting surface 72A and bone contacting surface 74A. Augment bore 180 also includes augment protrusion 182 extending from bore wall 181 into augment bore 180. Posterior augment 220 is mounted to condyle 40A on posterior side 38A of femoral component 210 such that femoral component contacting surface 72A is positioned adjacent posterior facet 58A of femoral component 210 and augment protuberances 86A contact posterior facet 58A. Once posterior augment 220 is properly aligned with posterior facet 58A, expansion collet 160 of condyle 40A is inserted into augment bore 180 such that fingers 164 of expansion collet 160 extend beyond augment protrusion 182 of posterior augment 220 as shown in Figs. 9A. With augment bore 180 of posterior augment 220 receiving expansion collet 160, longitudinal axis A₃ of augment bore 180 does not intersect anterior flange 36 (Fig. 5).

When collet fingers 164 are inserted into augment bore 180 of posterior augment 220, tapered exterior portion 168 of collet fingers 164 slide over and past augment protrusion 182 in augment bore 180 such that collet fingers 164 are located past augment protrusion 182 and augment protrusion 182 occupies annular groove 166. Fig. 9A illustrates collet fingers 164 in an unexpanded position, with collet fingers 164 slid past augment protrusion 182 and annular groove 166 positioned adjacent augment protrusion 182. Next, set screw 90A can be positioned in augment bore 180 and condyle bore 172. Set screw 90A defines fastener longitudinal axis A₄ and with set screw 90A positioned to secure posterior augment 220 to condyle 40A of femoral component 210, fastener longitudinal axis A₄ does not intersect anterior flange 36 (Fig. 5).

Once collet fingers 164 are in the position shown in Fig. 9A and set screw 90A positioned in augment bore 180 and condyle bore 172, socket head 104A of straight drive shaft 102A can be inserted into internal female socket 96A of set screw 90A, and set screw 90A can be tightened by rotating straight drive shaft 102A of torque wrench 100A using handle 106 (Fig. 4). As set screw 90A is threaded into condyle bore 172, tapered portion 94A of set screw 90A cooperates with tapered interior portion 170 of collet fingers 164 and pushes collet fingers 164 outward until exterior portion 168 of collet fingers 164 contact bore wall 181 of augment bore 180 and lock collet fingers 164 over augment protrusion 182 as shown in Fig. 9B. This configuration ensures that with collet fingers 164 mechanically locked over augment protrusion 182, posterior augment 220 is secured to posterior facet 58A of femoral component 210, such that, significant relative movement between augment 220 and femoral component 210 is prevented.

Referring to Figs. 7A and 7B, femoral knee prosthesis system 30 may include distal augment 120 secured to distal facet 56 of femoral component 31. Distal augment 120 provides structural support in areas of bone loss of a distal femur and prevents gaps between the distal femur and distal facet 56 of femoral component 31 installed on the distal femur. Distal augment 120 generally includes proximal surface 122, opposing distal surface 124, and distal augment cavity 126 spanning proximal surface 122 and distal surface 124. Distal augment cavity 126 includes internally threaded portion 128.

To secure distal augment 120 to distal facet 56 of femoral component 31, distal surface 124 of distal augment 120 is positioned atop distal facet 56 such that distal augment cavity 126 is positioned adjacent to distal bore 64 (shown in Fig. 2) located on distal facet 56 of femoral component 31. Distal bore 64 includes internally threaded bore portion 130. Upon properly positioning distal augment 120 to distal facet 56 and inserting fastener 132 having female socket 134 in distal augment cavity 126, a tool having a hexagonal cross-section can be inserted in female socket 134 of threaded fastener 132. The tool having a hexagonal cross-section can then be rotated to tighten threaded fastener 132 into threaded bore portion 130 to secure distal augment 120 to distal facet 56 of femoral component 31. Because no portion of femoral component 31 opposes distal facet 56, a variety of drivers including those having a straight drive shaft may be utilized to secure threaded fastener 132 in place as described above.

Distal augment 120 may be constructed of any biocompatible ceramic or metal, including, but not limited to, titanium or a titanium alloy, for example. Similar to posterior augment 70, distal augment 120 may also be constructed of a Tivanium™ material generally available from Zimmer, Inc. Distal augment 120 may also be constructed in whole or in part of a highly porous biomaterial such as a material produced using Trabecular Metal™ technology as described above.

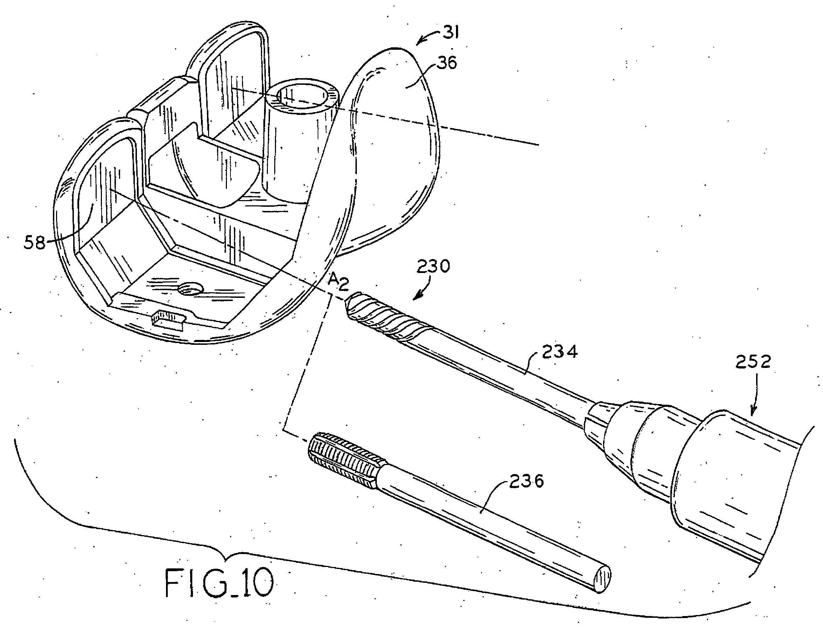

Referring to Fig. 10, bore 62 (Fig. 2) of femoral component 31 in accordance with the present disclosure may be produced using drill bit 230 to drill bore 62 in posterior facet 58 of femoral component 31. Because longitudinal axis A₂ of bore 62 does not intersect anterior flange 36, drill bit 230 can be positioned along a trajectory collinear with longitudinal axis A₂ to avoid anterior flange 36 and drill bore 62 in posterior facet 58. Advantageously, bore 62 of femoral component 31 is less costly to manufacture than conventional femoral knee prosthesis systems such as the one illustrated in Fig. 1. Referring to Fig. 1, femoral component 11 of conventional femoral knee prosthesis system 10 would not be able to have a bore (not shown) drilled in posterior side 24 using a drill bit because a drill bit would be unable to be positioned along a trajectory collinear with longitudinal axis A₁ because anterior flange 22 would block the drill bit from such a position. Thus, a more expensive and time consuming manufacturing technique such as electrical discharge machining (EDM) would be required to produce a bore in posterior side 24 of femoral component 11.

Referring to Fig. 2, during a manufacturing procedure to drill bore 62 in posterior facet 58 of femoral component 31, a location for bore 62 is determined in posterior facet 58 of femoral component 31, bore 62 having bore longitudinal axis A₂ that does not intersect anterior flange 36. Next, drill bit 230 (Fig. 10) having a drill bit width is selected for a rotary forming tool such as drill 232. Drive shaft 234 of the rotary forming tool includes a drive shaft periphery and a longitudinal axis and drive shaft 234 defines a drive shaft radius to the drive shaft periphery. Drive shaft 234 of the rotary forming tool is then positioned along a trajectory collinear with bore longitudinal axis A₂ such that with drill bit 230 positioned adjacent posterior facet 58 of femoral component 31, the longitudinal axis of drive shaft 234 does not intersect anterior flange 36 and is spaced from anterior flange 36 a minimum distance. The minimum distance is at least equal to the drive shaft radius to allow the drive shaft of the rotary forming tool to avoid anterior flange 36 when the drive shaft and the drill bit are used to form a bore through posterior facet 58 of femoral component 31. In one embodiment, the minimum distance is measured in a plane including anterior facet 52 (shown in Fig. 2). Finally, bore 62 is created by drilling a bore in posterior facet 58 of femoral component 31 using drill bit 230 positioned as described above. After bore 62 is formed, tap 236 may be utilized (e.g., together with drill 232) to thread bore 62. The drive shaft of tap 236 is sized so that it is spaced from anterior flange 36 in the same way that drive shaft 234 is spaced from anterior flange 36.

While this disclosure has been described as having exemplary designs, the present disclosure can be further modified within the scope of the invention as defined by the claims. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A femoral knee prosthesis component (31), comprising:
an anterior flange (36), said anterior flange having an anterior flange bone contacting surface comprising an anterior facet (52); and
a condyle (40, 42) extending from said anterior flange, said condyle having a condyle bone contacting surface comprising a facet (58) opposing said anterior facet (52) said condyle having a condyle bore (62) formed therein,
a posterior augment for securement to said condyle, said augment including an augment bore formed therethrough; and
a fastener sized and shaped to traverse said augment bore to engage said condyle and secure said posterior augment to said condyle, **characterised in that** the condyle bore defines a condyle bore longitudinal axis (A2) that intersects said facet and does not intersect said anterior flange.

2. The femoral knee prosthesis component of Claim 1, wherein said fastener comprises a threaded fastener, said threaded fastener sized and shaped to be alignable with said condyle bore longitudinal axis and securable relative to said posterior augment and the femoral knee prosthesis component by traversing said augment bore and said condyle bore to engage said posterior augment and said condyle, and secure said posterior augment to said condyle.

3. The femoral knee prosthesis component of Claim 2, wherein said fastener comprises a set screw (90), the femoral knee prosthesis component further comprises an expansion collet (80) and, said expansion collet having a threaded longitudinal bore, said set screw sized and shaped to threadingly engage said threaded longitudinal bore of said expansion collet and thereby actuate said expansion collet from an unexpanded state to an expanded state.

4. The femoral knee prosthesis component of Claim 3, wherein said expansion collet extends from said posterior augment, said condyle bore sized for receipt of said expansion collet when said expansion collet is in said unexpanded state, said condyle bore sized to cooperate with said expansion collet in said expanded state to secure said posterior augment to said condyle.

5. The femoral knee prosthesis component of Claim 3 or 4, wherein said expansion collet extends from said condyle, said augment bore sized for receipt of said expansion collet when said expansion collet is in said unexpanded state, said augment bore sized to cooperate with said expansion collet in said expanded state to secure said posterior augment to said condyle.

6. The femoral knee prosthesis component of any of claims 1 to 5, wherein said fastener defines a longitudinal axis and a peripheral extent and wherein said fastener further defines a fastener radius to said peripheral extent, said condyle bore longitudinal axis spaced a minimum distance from said anterior flange bone contacting surface, said minimum distance at least equal to said fastener radius.

7. The femoral knee prosthesis component of Claim 6, wherein said minimum distance is measured in a plane including said anterior facet.

8. A femoral knee prosthesis component as claimed in claim 1 and a driver (100), wherein said driver comprises:
a handle 106; and
a drive shaft (102) extending from said handle and terminating in a drive end, said drive shaft defining a longitudinal axis, said drive end matable with said fastener, said drive shaft defining a drive shaft radius from said longitudinal axis of said drive shaft to a perimeter extent of said drive shaft, with said drive shaft positioned to drive said fastener into an engaged position to secure said posterior augment to said condyle, said driver is rotatable through a rotation about said longitudinal axis of said drive shaft sufficient to actuate said fastener to secure said posterior augment to said condyle and said longitudinal axis of said drive shaft is positioned a minimum distance from said anterior flange throughout said rotation about said longitudinal axis of said drive shaft sufficient to actuate said fastener to secure said posterior augment to said condyle, said minimum distance at least equal to said drive shaft radius, whereby said drive shaft does not contact said anterior flange through said rotation about said longitudinal axis of said drive shaft sufficient to actuate said fastener to secure said posterior augment to said condyle.

9. The prosthesis and driver of Claim 8, wherein said minimum distance is measured in a plane including said anterior facet.

10. The prosthesis and driver of Claim 9, wherein said minimum distance is 4 mm.

11. A method of manufacturing a femoral prosthesis component (31) useable with a posterior augment (70), comprising:
forming a femoral prosthesis component of a biologically compatible material, the femoral prosthesis component comprising:
an anterior flange (36), the anterior flange having an anterior flange bone contacting surface; and
a condyle (40, 42) extending from the anterior flange, the condyle having a condyle bone contacting surface, the condyle bone contacting surface opposing the anterior flange bone contacting surface;
selecting a rotary forming tool, the rotary forming tool having a drive shaft with a drive shaft periphery and a longitudinal axis, the drive shaft defining a drive shaft radius to the drive shaft periphery;
positioning the rotary forming tool such that the longitudinal axis of the drive shaft of the rotary forming tool intersects the condyle bone contacting surface of the condyle at an intersection point and is oblique to said condyle bone contacting surface and the longitudinal axis of the drive shaft of the rotary forming tool is spaced a minimum distance from the anterior flange bone contacting surface, the minimum distance at least equal to the drive shaft radius, wherein, with the longitudinal axis of the drive shaft of the rotary forming tool intersecting said intersection point, the anterior flange presents a physical barrier preventing the rotary forming tool from being positioned perpendicular to the condyle bone contacting surface; and
with the rotary forming tool positioned as defined in said positioning step, actuating the rotary forming tool to form a bore through the condyle bone contacting surface and into the condyle.

12. The method of Claim 11, wherein the anterior flange bone contacting surface comprises an anterior facet (52) and wherein the minimum distance is measured in a plane including the anterior facet.

## Patentansprüche

1. Femorale Knieprothesekomponente (31), beinhaltend:
einen Anteriorflansch (36), wobei der Anteriorflansch eine
anteriorflansch-kochnenkontaktierende Fläche aufweist, eine Anteriorfacette (52) beinhaltend; und
einen Condylus (40, 42), der sich vom Anteriorflansch, erstreckt, wobei der Condylus eine condylus-kochnenkontaktierende Fläche aufweist, eine der Anteriorfacette (52) gegenüberliegende Facette (58) beinhaltend, wobei der Condylus eine darin geformte Condylus-Bohrung (62) aufweist,
ein Posterioraugment zur Sicherung des Condylus, wobei das Augment eine durch dieses geformte Augment-Bohrung aufweist; und
ein Befestigungselement, bemessen und geformt, um die Augment-Bohrung zu verschieben, um den Condylus in Eingriff zu bringen und das Posterioraugment am Condylus zu sichern, **dadurch gekennzeichnet, dass** die Condylus-Bohrung eine Condylus-Bohrung-Längsachse (A2) definiert, die die Facette schneidet und den Anteriorflansch nicht schneidet.

2. Femorale Knieprothesekomponente gemäß Anspruch 1, wobei das Befestigungselement ein mit einem Gewinde versehenes Befestigungselement beinhaltet, wobei das mit einem Gewinde versehene Befestigungselement bemessen und geformt ist, um posterior mit der Condylus-Bohrung-Längsachse ausrichtbar und sicherbar ist relativ zum Posterioraugment und der femoralen Knieprothesekomponente durch Verschieben der Augment-Bohrung und der Condylus-Bohrung, um das Posterioraugment und den Condylus in Eingriff zu bringen und das Posterioraugment am Condylus zu sichern.

3. Femorale Knieprothesekomponente gemäß Anspruch 2, wobei das Befestigungselement eine Stellschraube (90) beinhaltet und die femorale Knieprothesekomponente weiter einen Expansionsring (80) beinhaltet und der Expansionsring eine mit einem Gewinde versehene Längsbohrung aufweist, wobei die Stellschraube bemessen und geformt ist, um die mit einem Gewinde versehene Längsbohrung des Expansionsrings in Gewindeeingriff zu bringen und dabei den Expansionsrings von einem nicht expandierten Zustand in einen expandierten Zustand zu bringen.

4. Femorale Knieprothesekomponente gemäß Anspruch 3, wobei sich der Expansionsring vom Posterioraugment erstreckt, die Condylus-Bohrung für die Aufnahme des Expansionsrings bemessen ist, wenn sich der Expansionsring im nicht expandierten Zustand befindet, die Condylus-Bohrung bemessen ist, um mit dem Expansionsring im expandierten Zustand zusammenzuwirken, um das Posterioraugment am Condylus zu sichern.

5. Femorale Knieprothesekomponente gemäß Anspruch 3 oder 4, wobei sich der Expansionsring vom Condylus erstreckt, die Augment-Bohrung für die Aufnahme des Expansionsrings bemessen ist, wenn sich der Expansionsring im nicht expandierten Zustand befindet, die Augment-Bohrung bemessen ist, um mit dem Expansionsring im expandierten Zustand zusammenzuwirken, um das Posterioraugment am Condylus zu sichern.

6. Femorale Knieprothesekomponente gemäß einem der Ansprüche 1 bis 5, wobei das Befestigungselement eine Längsachse und eine periphere Ausdehnung definiert und wobei das Befestigungselement weiter einen Befestigungselementradius zur peripheren Ausdehnung definiert, wobei die Condylus-Bohrung-Längsachse eine Mindestdistanz von der anteriorflansch-kochnenkontaktierenden Fläche beabstanded ist, wobei die Mindestdistanz mindestens gleich dem Befestigungselementradius ist.

7. Femorale Knieprothesekomponente gemäß Anspruch 6, wobei die Mindestdistanz in einer Ebene gemessen wird, die die Anteriorfacette einschließt.

8. Femorale Knieprothesekomponente gemäß Anspruch 1 und ein Treiber (100), wobei der Treiber Folgendes beinhaltet:
einen Griff (106);
eine Treibwelle (102), die sich vom Griff erstreckt und in einem Treibende endet, wobei die Treibwelle eine Längsachse definiert, das Treibende mit dem Befestigungselement zusammengepasst werden kann, die Treibwelle einen Treibwellenradius von der Längsachse der Treibwelle zu einer Permiterausdehnung der Treibwelle definiert, wobei die Treibwelle positioniert ist, um das Befestigungselement in eine eingegriffene Position zu treiben, um das Posterioraugment am Condylus zu sichern, wobei der Treiber durch eine Rotation um die Längsachse der Treibwelle rotierbar ist, ausreichend, um das Befestigungselement zu betätigen, um das Posterioraugment am Condylus zu sichern, und die Längsachse der Treibwelle eine Mindestdistanz vom Anteriorflansch während der ganzen Rotation um die Längsachse der Treibwelle positioniert ist, ausreichend, um das Befestigungselement zu betätigen, um das Posterioraugment am Condylus zu sichern, wobei die Mindestdistanz mindestens gleich dem Treibwellenradius ist, wobei die Treibwelle den Anteriorflansch durch die Rotation um die Längsachse der Treibwelle nicht kontaktiert, ausreichend, um das Befestigungselement zu betätigen, um das Posterioraugment am Condylus zu sichern.

9. Prothese und Treiber gemäß Anspruch 8, wobei die Mindestdistanz in einer Ebene gemessen wird, die die Anteriorfacette einschließt.

10. Prothese und Treiber gemäß Anspruch 9, wobei die Mindestdistanz 4 mm beträgt.

11. Verfahren zum Herstellen einer femoralen Knieprothesekomponente (31), die mit einem Posterioraugment (70) verwendet werden kann, beinhaltend:
Formen einer femoralen Knieprothesekomponente aus einem biologisch verträglichen Material, die femorale Knieprothesekomponente Folgendes beinhaltend:
einen Anteriorflansch (36), wobei der Anteriorflansch eine
anteriorflansch-kochnenkontaktierende Fläche aufweist; und
einen Condylus (40, 42), der sich vom Anteriorflansch, erstreckt, wobei der Condylus eine condylus-kochnenkontaktierende Fläche aufweist, wobei die condylus-kochnenkontaktierende Fläche der anteriorflansch-kochnenkontaktierenden Fläche gegenüberliegt;
Auswählen eines Rotationsformwerkzeugs, wobei das Rotationsformwerkzeug eine Treibwelle mit einer Treibwellenperipherie und einer Längsachse aufweist, wobei die Treibwelle einen Treibwellenradius zur Treibwellenperipherie definiert;
Positionieren des Rotationsformwerkzeugs, so dass die Längsachse der Treibwelle des Rotationsformwerkzeugs die condylus-kochnenkontaktierende Fläche des Condylus am Schnittpunkt schneidet und schräg zur condylus-kochnenkontaktierenden Fläche liegt und die Längsachse der Treibwelle des Rotationsformwerkzeugs eine Mindestdistanz von der anteriorflansch-kochnenkontaktierenden Fläche beabstandet ist, wobei, mit der Längsachse der Treibwelle des Rotationsformwerkzeugs den Schnittpunkt schneidend, der Anteriorflansch eine physische Barriere darstellt, die verhindert, dass das Rotationsformwerkzeug senkrecht zur condylus-kochnenkontaktierenden Fläche positioniert wird; und
mit dem Rotationsformwerkzeug, wie im Positionierungsschritt positioniert, Betätigen des Rotationsformwerkzeugs, um eine Bohrung durch die condylus-kochnenkontaktierende Fläche und in den Condylus zu formen.

12. Verfahren gemäß Anspruch 11, wobei die anteriorflansch-kochnenkontaktierende Fläche eine Anteriorfacette (52) beinhaltet und wobei die Mindestdistanz in einer Ebene gemessen wird, die die Anteriorfacette einschließt.

## Revendications

1. Un composant fémoral de prothèse du genou (31), comprenant :
unebride antérieure (36), laditebride antérieure ayant une surface de contact osseux de bride antérieure comprenant une facette antérieure (52) ; et
un condyle (40, 42) partant de laditebride antérieure, ledit condyle ayant une surface de contact osseux de condyle comprenant une facette (58) opposée à ladite facette antérieure (52), ledit condyle ayantun alésage de condyle (62), formé dans cet élément,
une augmentation postérieure pour la fixation dudit condyle, ladite augmentation incluant un alésage d'augmentation en son travers ; et
un élément de fixation calibré et façonné pour traverser ledit alésage de l'augmentation pour engager ledit condyle et fixer ladite augmentation postérieure audit condyle, **caractérisé en ce que** l'alésage du condyle définit un axe longitudinal d'alésage du condyle (A2) qui croise ladite facette et ne croise pas ladite bride antérieure.

2. Le composant fémoral de prothèse du genou de la Revendication 1, où ledit élément de fixation comprend un élément de fixation fileté, ledit élément de fixation fileté calibré et façonné pour être en position postérieure et alignable par rapport audit axe longitudinal d'alésage du condyle et être fixable par rapport à ladite augmentation postérieure et le composant fémorale de prothèse du genou en traversant ledit alésage de l'augmentation et ledit alésage du condyle pour engager ladite augmentation postérieure et ledit condyle, et fixer ladite augmentation postérieure audit condyle.

3. Le composant fémoral de prothèse du genou de la Revendication 2, où ledit élément de fixation comprend une vis de pression (90) et le composant fémoral de prothèse du genou comprend également un collet d'expansion (80) et, ledit collet d'expansion ayant un alésage longitudinal fileté, ladite vis de pression étant calibrée et façonnée pour s'engager par un filetage dans ledit alésage longitudinal fileté dudit collet d'expansion et actionner ainsi ledit collet d'expansion d'un étant non déployé à déployé.

4. Le composant fémoral de prothèse de genou de la Revendication 3, où ledit collet d'expansion se déploie depuis ladite augmentation postérieure, ledit alésage du condyle étant calibré pour recevoir ledit collet d'expansion lorsque ledit collet d'expansion est dans ledit état non déployé, ledit alésage du condyle étant calibré pour coopérer avec ledit collet d'expansion dans ledit état déployé pour fixer ladite augmentation postérieure audit condyle.

5. Le composant fémoral de prothèse de genou de la Revendication 3 ou 4, où ledit collet d'expansion se déploie depuis ledit condyle, ledit alésage de l'augmentation étant calibré pour recevoir ledit collet d'expansion lorsque ledit collet d'expansion est dans ledit état non déployé, ledit alésage de ladite augmentation étant calibré pour coopérer avec ledit collet d'expansion dans ledit état déployé pour fixer ladite augmentation postérieure audit condyle.

6. Le composant fémoral de prothèse du genou des revendications 1 à 5, où ledit élément de fixation définit un axe longitudinal et une portée périphérique et où ledit élément de fixation définit également un rayon de l'élément de fixation par rapport à ladite portée périphérique, ledit axe longitudinal d'alésage du condyle étant espacé d'une distance minimale de ladite surface de contact osseux de la bride antérieure, ladite distance minimale étant au moins égale audit rayon de l'élément de fixation.

7. Le composant fémoral de la prothèse du genou de la Revendication 6, où ladite distance minimale est mesurée dans un plan incluant ladite facette antérieure.

8. Un composant fémoral de prothèse du genou selon la revendication 1 et une commande (100), où ladite commande comprend :
une poignée (106) ; et
un arbre de transmission (102) partant de ladite poignée et se terminant sur une extrémité de transmission, ledit arbre de transmission définissant un axe longitudinal, ladite extrémité de transmission pouvant être assemblée avec ledit élément de fixation, ledit arbre de transmission définissant un rayon d'arbre de transmission depuis ledit axe longitudinal dudit arbre de transmission vers un périmètre de portée dudit arbre de transmission, avec ledit arbre de transmission positionné pour mettre ledit élément de fixation dans une position engagée afin de fixer ladite augmentation postérieure audit condyle, ladite commande est rotative via une rotation sur ledit axe longitudinal dudit arbre de transmission suffisamment pour actionner ledit élément de fixation pour fixer ladite augmentation postérieure audit condyle et ledit axe longitudinal dudit arbre de transmission est positionné à une distance minimale de ladite bride antérieure via ladite rotation sur ledit axe longitudinal dudit arbre de transmission suffisamment pour actionner ledit élément de fixation pour fixer ladite augmentation postérieure audit condyle, ladite distance minimale étant au moins égale audit rayon d'arbre de transmission, par lequel ledit arbre de transmission n'entre pas en contact avec ladite bride antérieure via ladite rotation sur ledit axe longitudinal dudit arbre de transmission suffisamment pour actionner ledit élément de fixation pour fixer ladite augmentation postérieure audit condyle.

9. La prothèse et la commande de la Revendication 8, où ladite distance minimale est mesurée dans un plan incluant ladite facette antérieure.

10. La prothèse et la commande de la Revendication 9, où ladite distance minimale est de 4 mm.

11. Un procédé de fabrication d'un composant fémoral de prothèse (31) utilisable avec une augmentation postérieure (70) comprenant :
la formation d'un composant fémoral de prothèse fabriqué dans un matériau biologiquement compatible, le composant fémoral de prothèse comprenant :
une bride antérieure (36), la bride antérieure ayant une surface de contact osseux de la bride antérieure ; et
un condyle (40, 42) partant de la bride antérieure, le condyle ayant une surface de contact osseux du condyle, la surface de contact osseux du condyle étant opposée à la surface de contact osseux de la bride antérieure ;
la sélection d'un outil de formation rotatif, l'outil de formation rotatif ayant un arbre de transmission avec une périphérie d'arbre de transmission et un axe longitudinal, l'arbre de transmission définissant un rayon d'arbre de transmission par rapport à la périphérie de l'arbre de transmission ;
le positionnement de l'outil de formation rotatif de sorte que l'axe longitudinal de l'arbre de transmission de l'outil de formation rotatif croise la surface de contact osseux du condyle à un point d'intersection et soit en oblique par rapport à ladite surface de contact osseux du condyle et l'axe longitudinal de l'arbre de transmission de l'outil de formation rotatif soit à une distance minimale de la surface de contact osseux de la bride antérieure, la distance minimale étant au moins égale au rayon de l'arbre de transmission, où, avec l'axe longitudinal de l'arbre de transmission de l'outil de formation rotatif croisant ledit point d'intersection, la bride antérieure constitue une barrière mécanique empêchant l'outil de formation rotatif de se positionner à la perpendiculaire de la surface de contact osseux du condyle ; et
avec l'outil de formation rotatif positionné comme indiqué à ladite étape de positionnement, il actionne l'outil de formation rotatif pour former un alésage au travers de la surface de contact osseux du condyle et dans le condyle.

12. Le procédé de la Revendication 11, où la surface de contact osseux de la bride antérieure comprend une facette antérieure (52) et où la distance minimale est mesurée dans un plan incluant la facette antérieure.
